# EUROPEAN PATENT APPLICATION

(11) **EP 2 799 098 A1**
(43) Date of publication of application: **05.11.2014**
(21) Application number: 13165751.2
(22) Date of filing: 29.04.2013
(51) Int. Cl.: A61M 5/142, A61M 5/145, A61M 5/172

(54) **Ambulatory infusion system including wireless earphone device**

(71) Applicant: F.Hoffmann-La Roche AG, 4070 Basel (CH); Roche Diagnostics GmbH, 68305 Mannheim (DE)
(72) Inventor: The designation of the inventor has not yet been filed
(74) Representative: Rentsch Partner AG

(57) **Abstract**

Disclosed is an ambulatory infusion system, including:
- an infusion pump device (1) including a pump unit (11) and an electronic pump control unit (12), the pump control unit (12) being operatively coupled to the pump unit (11) to control the pump unit (11) to continuously infuse a liquid drug into a patient's body, the infusion pump device (1) further including a wireless pump communication unit (15), wherein the infusion pump device (1) is designed to generate a pump message to be signalized to a user, and
- a signalization device for providing user signalization in accordance with the pump message,
wherein the signalization device includes an earphone device (2), the earphone device (2) including an earphone audio transducer (22), the earphone audio transducer (22) being designed for attachment to a user's head in direct acoustic coupling to an ear of the user, the earphone device (2) further including a wireless earphone communication unit (21),

wherein the infusion pump device (1) is designed to transmit the pump message, via the pump communication unit (15), to the earphone communication unit (21), and wherein the earphone device (2) is designed to receive the pump message via the earphone communication unit (21) and to actuate the earphone audio transducer (22) to produce a pump audio message in accordance with the pump message.

## Description

### Technical field

The present disclosure lies in the technical field of ambulatory infusion systems and ambulatory infusion pumps. Disclosed are further methods for providing audio messages to a user of such systems and pumps.

Embodiments in accordance with the present disclosure may be used in a number of applications and are of particular use in the field infusion systems for diabetes therapy.

### Background and prior art

Continuous Subcutaneous Insulin Infusion (CSII) is an established and therapeutical advantageous way of treating diabetes mellitus. In CSII, a diabetic carries a miniaturized infusion pump substantially continuously, night and day. The infusion pump infuses minimal quantities of insulin in a substantially continuous way according to a person-specific, generally time-variable infusion schedule, thus providing a so-called basal amount of insulin that is required by the diabetic's body for maintaining a normal or close-to-normal metabolism and in particular blood glucose concentration. For typical state-of-the-art systems, the basal administration schedule follows a generally circadian cycle and is preset by a healthcare professional. In addition, insulin pumps are designed to administer larger insulin quantities, so called boli, within a short period of time on demand, in order to cover diabetic's food intake, and under exceptional circumstances, such as illness or in case of a hyperglycaemia (also referred to as hyperglycaemic excursion or hyperglycaemic episode), which is a situation of increased blood glucose concentration, resulting from a relative lack of insulin within the diabetic's body.

In use, various situations occur where interaction is required between the device and a device user, such as a diabetic, or a relative like parent, a teacher, or any other person involved in the therapy management. Those situations, include, e.g., the programming of boli for a meal, replacing disposables such as batteries, drug cartridges and infusion lines, reviewing therapy history data that are store in a device memory, and the like.

For most of those interactions, the device generates feedback that is provided by the device to the user. For this purpose, the device typically includes a miniaturized pump audio transducer, and a non-audio indication unit, typically a display. In addition, safety-critical indications are provided in case of situations that require immediate awareness and typically immediate user interaction, such as an empty reservoir or battery or a leakage or blockage of the infusion line.

With increasing use of ambulatory infusion systems, there is an increasing demand for the systems to be carried and operated in a discrete way and without drawing any attention, e.g. in the public or business life.

To meet this demand at least to some extent, devices have been developed that include a pager vibrator as non-audio indication unit that is used for providing user feedback during general device interaction as well as for non-critical information and notification purposes. Safety-critical messages, however, are generally provided, exclusively or additionally, via the pump audio transducer. In addition, the information that can be provided via pager vibrator is comparatively limited.

The WO 2009/ 0776443 A2 discloses a portable infusion pump device that includes a media player for playing back music or other media content via an earbud device, earphone device, or the like. Playback of media contents may be automatically interrupted to deliver alarm or alert messages. The WO 2009/ 081403 A2 discloses an insulin infusion device with earphones for providing information, e.g. with respect to physical exercise of the user.

It is a general object of the present disclosure to improve the state of the art with respect to providing audio feedback and message to a user of an ambulatory infusion system in a discrete way.

### Summary of disclosure

According to a first aspect, the present disclosure is directed towards an ambulatory infusion system.

An ambulatory infusion system in accordance with the present disclosure may include an infusion pump device including a pump unit and an electronic pump control unit, the pump control unit being operatively coupled to the pump unit to control the pump unit to continuously infuse a liquid drug into a patient's body. The infusion pump device may further include a wireless pump communication unit and may be designed to generate a pump message to be signalized to a user.

The ambulatory infusion device may further include a signalization device for providing user signalization in accordance with the pump message. The signalization device may include an earphone device, the earphone device including an earphone audio transducer. The earphone audio transducer may be designed for attachment to a user's head in direct acoustic coupling to an ear of the user. The earphone device may further include a wireless earphone communication unit, wherein the infusion pump device is designed to transmit the pump message, via the pump communication unit, to the earphone communication unit. The earphone device may be designed to receive the pump message via the earphone communication unit and to actuate the earphone audio transducer to produce a pump audio message in accordance with the pump message.

Besides meeting the demand for providing signalization in a discrete way, a system in accordance with the present disclosure may be favourably used in scenarios where the diabetic who carries the infusion pump device is not identical with the person who is acting as "user" and should accordingly receive the signalizations, alternatively or additionally to the diabetic. Such a scenario is given for example, if the diabetic is a small infant and the user operating the system is a parent, a responsible nanny, teacher, or the like.

In some embodiments, the ambulatory infusion system may further include at least one further device, the at least one further device including a wireless further device communication unit, the further device communication unit being designed for wireless coupling, in particular direct wireless coupling, with at least one of the pump communication unit and the earphone communication unit.

Including into the system -, in addition to the infusion pump device and the earphone device - further devises allows a number of favourable embodiments with respect to system design, user comfort and discreetness as will be discussed further below both in the general description of the disclosure and in the context of exemplary embodiments.

Without excluding other types of further devices, the at least one further device may include either of a cell phone, a pump remote controller, a blood glucose measurement device, and a continuous blood glucose monitor.

In embodiments where a number of further devices is present, they may be realized by a number of physically distinct and separated devices or be, fully, or partly, be realized by functional units within a common physical unit. For example, a smart phone may include the functionality of a pump remote controller or a pump remote controller may include a blood glucose measurement device.

In some embodiments, the further device communication unit is designed to receive the pump message from the pump communication unit and to relay the pump message to the earphone communication unit.

In some embodiments including at least one further device, the at least one further device is designed to generate at least one further device message and to transmit the at least one further device message, via the further device communication interface, to the pump communication interface, and the pump communication interface is designed to receive the at least one further device message and to relay the at least one further device message to the earphone communication interface.

The at least one further device message may include any or more of the different types of pump messages as discussed above, i.e. user feedback during operation, status information, safety-critical alert or alarm messages, etc.

Alternatively or additionally, the at least one further device is, in some embodiments, designed to transmit data, via the further device communication interface, to the pump communication interface and the infusion pump device is designed to receive, via the pump communication interface, the data and to generate the at least one further device message based on the transmitted data, and to transmit the at least one further device message, via the pump communication interface, to the earphone communication interface.

These data may be any data on which a further device message may be based, such as a battery charging status, a measured blood glucose value or trend, or a warning, alarm or alert message relating to operation of the at least one further device.

In contrast to the before-described option where a further device message is generated by the at least one further device and the infusion pump device mainly serves as relay, this type of embodiment requires and involves processing of the data and generation of the further device message by the infusion pump device.

In some embodiments, the earphone device includes an earphone input unit, wherein the pump communication unit and the earphone communication unit are designed for transmission of a user input that is entered via the earphone input unit from the earphone device to the infusion pump device.

The earphone input unit may generally be realized, alone or in combination, by any input unit known in the art, such as pushbuttons, a touch pad or a miniaturized microphone in combination with a voice recognition unit. This type of embodiment allows providing user input in a discreet and convenient way, e.g. when the infusion pump is carried concealed from view and not easy accessible, e.g. as a patch directly attached to the skin at the infusion site, as necklace, etc.

In some embodiments including an earphone input unit, the user input includes an information request input, and the pump control unit is designed to generate the pump message in response to receiving, via the pump communication unit, the information request input.

An information request input is a user request for receiving device- or therapy related information, such as the time of day, the current amount of drug in the drug container, the time and/ or amount of the last ordered bolus, or the like. This type of information may be requested by the user (user-operation) for information purposes and is not related to critical warnings, alarms or alerts that may be are generated by the system at any time (device-operation).

In some embodiments, the signalization device includes a non-audio indication unit and the infusion pump device is designed to actuate the non-audio indication unit in accordance with the pump message additionally or alternatively to transmitting the pump message to the earphone communication unit.

A non-audio indication unit may especially include a display and/ or a pager vibrator.

In some embodiments, the infusion pump device is designed to generate the pump message alternatively as a number of distinct pump messages.

The distinct pump messages may generally include any type of critical and/ or non-critical messages as explained before and may include any or both of user-pull and device-push messages.

In some embodiments including a number of distinct pump messages, each of the number of distinct pump messages is associated with a corresponding signalization status that may be independently activated or deactivated, with an activated signalization status indicating that a signalisation is to be provided in accordance with the corresponding pump message. Each of the number of distinct pump messages is associated with a corresponding signalization priority, and the ambulatory infusion system is designed to determine, within a number of pump messages having an activated associated signalization status, a priority pump message of highest signalization priority, and to control the signalization device to provide user signalization in accordance with the priority pump message.

Favourably, non-critical information messages, such as an information that the remaining drug volume has fallen below some threshold are associated with a low signalization priority, while messages that inform a bout a situation that require soon or immediate action are associated with a high signalization priority. The number of different alerting priorities may be two or more.

In some embodiments, the infusion pump device includes a pump audio transducer and the pump control unit is designed to actuate the pump audio transducer in accordance with the pump message additionally or alternatively to transmitting the pump message to the earphone communication unit.

In some embodiments including a pump audio transducer, the pump control unit is designed to selectively actuate the pump audio transducer in accordance with the pump message based on the pump message priority associated with the pump message.

This type of embodiment ensures that messages of high signalization priority, such as safety-critical messages, are indicated directly via the pump audio transducer, independent of factors such as whether or not an operative communication channel to the earphone device is or can be established, whether or not the user actually carries the earphone device, and the like. Those high priority messages may or may not be additionally transmitted to the earphone device. Low priority messages, such as status information messages and user feedback, in contrast, are only transmitted to and indicated by the earphone device and not the pump audio transducer for discreetness reasons.

In some embodiments including a pump audio transducer, the infusion pump device is designed to determine whether a communication channel between the pump communication unit and the earphone communication unit is established and to transmit the pump message to the earphone communication unit without activating the pump audio transducer if communication between the pump communication unit and the earphone communication unit is established and to activate the pump audio transducer if communication between the pump communication unit and the earphone communication unit is not established.

This type of embodiment ensures that an indication is provided to the user for a pump audio message, whether or not a communication channel between pump communication unit and earphone communication unit is established. It is, however, particularly discrete because it ensures that indication is provided via the earphone device if possible, i.e., if a working communication channel is established and the less discrete pump audio transducer is used only where this requirement is not fulfilled, e.g. because the earphone device is out of range, defective or simply switched off.

In some embodiments including a pump audio transducer, the infusion pump device is designed to transmit the pump message, via the pump communication unit, to the earphone communication unit without activating the pump audio transducer and to activate the pump audio transducer if no acknowledgement user input is received within a following acknowledgement time interval.

For this type of embodiment, it is first tried to provide an indication via the earphone device, and the pump audio transducer is only activated in a second step if indication of the pump audio message via the earphone device is not acknowledged, e.g. because of the user having a nap, being distracted or simply not taking notice because of ambient noise. While the pump audio transducer may optionally be activated anyway for safety reasons, this type of embodiment generally allows to provide critical messages, e.g. high-priority messages as discussed above, exclusively in a desecrate way via the earphone device where possible, without the risk of the message being finally lost or actually received by the user too late. A typical acknowledgement time interval may, e.g., be in a range of 30 sec to 2 min.

For embodiments including a pump audio transducer, it should be noted that different strategies for providing a pump audio message may be applied for different messages, in dependence, e.g., on the alerting priority. For a high priority message, for example, the pump audio transducer may be activated in any case; for a medium priority message, the pump audio transducer may be activated only if no acknowledgement user input is received within an acknowledgement time period, while a low-priority message may in any case be provided via the earphone device only, independent on whether it is actually received and taken notice off by the user.

In some embodiments further including an earphone input unit, a user input that may be entered via the earphone input unit includes the acknowledgement user input.

In some embodiments, the ambulatory infusion system includes a voice message unit, the voice message unit being designed to receive the pump message and to generate, based on the pump message, a corresponding pump voice message, and to actuate the earphone audio transducer to produce the pump voice message.

In some embodiments including a voice message unit, the voice message unit is included in either of the infusion pump device or the earphone device.

Alternatively, however, the voice message unit may be implemented in a further device, such as a smart phone.

In some embodiments including a voice message unit, the pump voice message is indicative of a situation requiring urgent user interaction, in particular a blockage or leakage of an infusion line, an empty liquid drug container, an empty power source, or a electric and/ or mechanic failure of the ambulatory infusion system.

In some embodiments, the signalization device further includes a head-mounted display apparatus and the infusion pump device is designed to generate at least one further pump message and to transmit the at least one further pump message, via the pump communication unit, to the head-mounted display apparatus. The head-mounted display apparatus is designed to receive the wirelessly transmitted further pump message and to actuate an image-forming device of the head-mounted display device to produce a pump display message in accordance with the at least one further pump message.

The head-mounted display device is designed to generate, via the image-forming device, a virtual image that may be observed by the user carrying the device.

While a variety of designs is possible and known in the art, the head-mounted display device may, in a typical embodiment, have the form of glasses. The head-mounted display device may, e.g. be designed in accordance with the disclosure of the US 2013/ 0069850 A1 and/ or the prior art cited therein.

The head-mounted display device may be distinct and physically separate device or may be formed in an integral way with the earphone device as common compact unit.

Furthermore, the earphone device and the head-mounted display device may share common units, such as a power supply. The head-mounted display device may wirelessly receive the further pump message via a dedicated head mounted display communication unit or via the earphone communication unit.

The at least one further pump message may be identical to the pump message that is indicated by the earphone device or may be a separate message.

This type of embodiment allows, for example to provide a warning, alarm or alert both as pump audio message and also provide corresponding plain-text information, such as an alarm message text and optionally further related and useful information, such as proposed handling steps to be taken by the user.

The head-mounted display may also be used to provide information that is not related to pump audio messages in a both comfortable and discrete way, such as the date and time of day, status information like the current drug cartridge content or an infusion system user manual.

In an analogue way as described above and further below in context of the earphone device, the head-mounted display apparatus may, in addition to messages generated by the infusion pump device, display further device messages that origin from further devices of the ambulatory infusion system.

According to a further aspect, the present disclosure is directed towards an infusion pump device that is designed for use in an ambulatory infusion system according to any of the preceding claims.

According to a still further aspect, the present disclosure is directed towards a method for providing signalizations to a user of an ambulatory infusion system. The method may include generating, by an infusion pump device, a pump message to be signalized to the user, transmitting the pump message to an earphone device, and actuating an earphone audio transducer of the earphone device to produce a pump audio message in accordance with the pump message.

It has to be understood that infusion pump devices may especially be part of an ambulatory infusion system according to the present disclosure; the method for providing signalizations may be carried out by an ambulatory infusion system and an ambulatory infusion pump device according to the present disclosure. Therefore, specific embodiments of the ambulatory infusion system define, at the same time, corresponding embodiments of an infusion pump device and vice versa. Similarly, specific embodiments of the method for providing signalization define, at the same time, corresponding embodiments of an ambulatory infusion system and an infusion pump device, and vice versa.

### Exemplary embodiments

In the following, exemplary embodiments in accordance with the present disclosure are described in more detail with reference to the figures. Between the figures, elements having substantially identical or corresponding function or design generally have the same reference numbers.
Figure 1, Figure 2 each show an exemplary ambulatory infusion system in a schematic structural view.
Figure 3a-3d schematically shows a further exemplary ambulatory infusion system including a number of separate devices and their communication for providing signalization.
Figure 4, Figure 5 each show simplified operational flows for providing signalization via an earphone device.

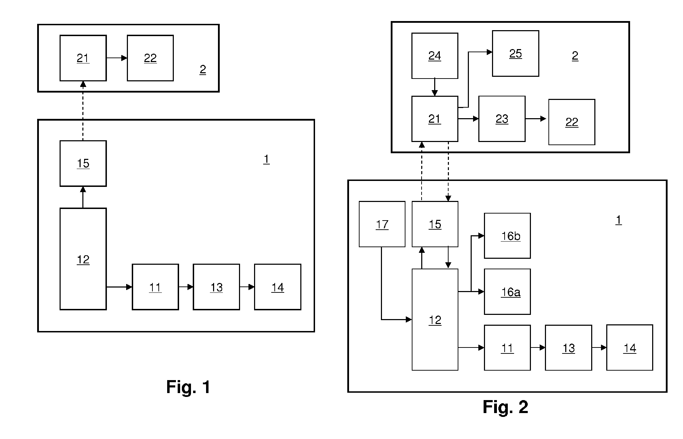

Figure 1 shows an exemplary ambulatory infusion system in a schematic structural view, the ambulatory infusion system including an infusion pump device 1 and an earphone device 2.

Like the following figures, Figure 1 only shows functional components that relate to the present disclosure, without excluding the optional presence of further structural and/ or functional elements or units. In the figures, solid lines or arrows indicated a typically hardwired or galvanic coupling, while dashed lines arrows or arrows indicate an operative coupling that is typically realized as non-galvanic coupling.

Infusion pump device 1 includes a pump unit 11, a pump control unit 12, a liquid drug cartridge 13, an infusion line 14, and a pump communication unit 15.

Earphone device 2 includes an earphone communication unit 21, an earphone and an earphone audio transducer 22.

Pump unit 11 is in the following exemplarily considered as being a syringe driver pump with a motor-driven threaded spindle. The spindle acts on a piston of a liquid drug container 13, the piston being sliding and sealing arranged in a barrel-like container body. In operation, liquid drug is forced, by displacing the piston, out of an outlet of the container body and into infusion line 14.

Operation of pump unit 11 is controlled and supervised by pump control unit 12. Pump control unit 12 is realized by microcontroller-based solid state circuitry as generally known in the art. Pump control unit 12 drives and operates pump unit 11 in accordance with a typically time-variable infusion schedule that may be stored in a memory of control unit 12. Control unit 12 may additionally control the infusion of liquid drug boli on demand.

Wireless pump communication unit 15 is operatively coupled to pump control unit 12 to receive a pump message for transmission to corresponding wireless earphone communication unit 21. Pump communication unit 15 and earphone communication unit 21 may be designed according to various technologies, e.g. infra-red (IR). Typically, however, pump communication unit 15 and earphone communication unit 21 are designed for radio frequency (RF) communication either according to a proprietary or a general-purpose communication standard, such as Bluetooth®.

When receiving a pump message from pump communication module 15, earphone communication unit 21 actuates earphone audio transducer 22. Further dedicated circuitry of earphone device 2, such as driver circuitry for driving earphone audio transducer 22, is not shown in Figure 1 for clarity reasons, but may be present as well.

Both infusion pump device 1 and earphone device 2 typically include a power supply (not shown), e.g. in form of a rechargeable or non-rechargeable battery, and may further include additional elements and functional units, such as power management circuitry, a display, safety circuitry, etc. Pump unit 12 may include sensors, such as one or more of a rotatory motor encoder and/ or a force sensor to measure the force that is exerted by the spindle onto the piston of container 13, the fluidic pressure in container 12 and/ or infusion line 13, a liquid flow sensor etc. Signals provided by these and other sensors are evaluated by corresponding hardware and/ or firmware components in order to detect situations that require a user signalization via a corresponding pump message. A pump message may be of any type as discussed above in the context of the general the disclosure. It may inform the user about an expected or upcoming situation that will require user interaction, such as a battery powering the infusion pump device or the drug container being almost empty, i.e. a state of the system that will result in a critical situation if not taken care off within a certain time frame. A pump message may further inform the user about an existing safety-critical situation that requires urgent interaction, such as a blockage or leakage of infusion line 14, an empty power source or liquid drug container, or an electrical or mechanical failure. The pump message may further be a pure information message and indicate the user, e.g. of the current content of container 13, the date of the last bolus infusion, or the like.

The pump message may be directly generated as audio message by infusion pump device 1 and transmitted to earphone device 2 in any suited analogue or digital form. Alternatively, the pump message may be generated in form of a digital code that is transmitted to earphone device 2.

Figure 2 shows another embodiment of an exemplary ambulatory infusion system. In the following, only those aspects are discussed that are present in addition to or a realized in a substantially different way as compared to the embodiment of Figure 1.

In the embodiment of Figure 2, infusion pump device 1 includes a non-audio indication unit 16a which may especially include one or optical indication devices as known in the art, such as graphic or non-graphic displays, or (organic) light emitting diodes (O)LEDs. Non-audio indication unit 16a may further include a tactile indication device, such as a pager vibrator.

In the embodiment of Figure 2, infusion pump device 1 further includes optional pump audio transducer 16b, typically realized as buzzer, or miniaturized loudspeaker.

In the embodiment of Figure 2, infusion pump device 1 additionally includes optional pump input unit 17 in operative coupling to pump control unit 12. Pump input unit 17 may include one or a number of pushbuttons, a touch screen, an acoustic input unit that is designed to receive and process voice commands, etc. For exemplary purposes, pump input unit 17 may be considered as a number of push buttons.

Pump input unit 17 together with non-audio indication unit 16a and/ or pump audio transducer 16b are favourably used for general operation of the infusion system, including operations such as programming a basal infusion schedule, initiating the administration of on-demand drug boli at mealtime, reviewing a stored therapy and device history, and the like. Non-audio indication unit 16a and pump audio transducer 16b may, additionally or alternatively, provide user signalization in accordance with pump messages in addition or alternatively to earphone device 2. A more detailed discussion of these and related aspects is given further below.

In contrast to the embodiment of Figure 1, pump communication unit 15 and earphone communication 21 are designed for bidirectional communication in the embodiment of Figure 2. Bidirectional communication, as generally present, e.g., according to the Bluetooth® standard, enables safe and convenient pairing between infusion pump device 1 and earphone device 2. Furthermore, integrity of the communication link between pump communication unit (15) and earphone communication unit 21 may be checked continuously or in appropriate time intervals and allows verifying correct data transmission between pump communication unit 15 and earphone communication unit 21.

In the embodiment of Figure 2, earphone device 2 further includes optional earphone input unit 24 in operative coupling with earphone communication unit 21. Similar, to pump input unit 17, a variety of technologies may be used for realizing earphone input unit 24. Favourably, earphone input unit 27 is optimized to be small in dimensions, lightweight and allow discrete operation. Earphone input unit 24 may, e.g. be realized by a single miniaturized push button or a touch-sensitive capacitive switch.

Earphone input unit 24 may be used to input operational commands for infusion pump device 24, such as the programming of a meal bolus. In this way, essential operations may be inputted in a discrete way, as highly desired, e.g., when administering a meal bolus in a public environment such as a restaurant, without having to physically access pump input unit 17 or any other bulky control device. Earphone input unit 24 may further be used for requesting information from infusion pump device 1 for acoustic indication via earphone unit 2. Typical examples for information that may be requested are a battery status or the filling level of drug container 14.

If used for requesting information from infusion pump device 1, a command indicating an actuation of earphone input unit 24 is transmitted, via communication link 21 , 15, to pump control unit 12 where it is processed and a corresponding pump message is generated in response. The pump message is transmitted back to earphone device 2 and earphone audio transducer 22 is actuated in accordance with the pump message

Earphone input unit 24 may further be used for muting and/ or cancelling indications that are provided via one or more of earphone audio transducer 22, non-audio indication unit 16a and pump audio transducer 16b.

In the embodiment of Figure 2, earphone device 2 further includes optional voice message unit 23 in operative coupling to earphone communication unit 21 and earphone transducer 22. Voice message unit 23 may store readily recorded voice messages and/ or may be designed to synthesize situation-specific voice messages. Readily recorded voice messages are especially suited for providing pre-defined and "static information" , such as the occurrence of one of a limited number of situations that require user interaction. Synthesized voice messages are especially suited for providing non-predefined information, such as a drug amount remaining in drug container 13 or a bolus amount that has been programmed for administration. Furthermore, a combination is possible where a voice message is partly pre-defined while other parts of the message are synthesized. For indicating the container content, a voice message may, e.g. be "The current container content is 271 Units". In this message, "271" may be synthesized and the rest of the message may be pre-recorded.

Alternatively to earphone device 2, voice message module 23 may, if present, be realized integral with infusion pump device 1.

In the embodiment of Figure 2, an optional head-mounted display apparatus 25 is additionally present. While head-mounted display apparatus 25 may alternatively be a distinct and physically separate device, it is shown as integral part of earphone device 2. In this embodiment, head-mounted display apparatus 25 is operatively coupled to earphone communication unit 21 and receives the at least one pump message as previously discussed via earphone communication unit 21.

While on shown in the specific embodiment of Figure 2, a head-mounted display apparatus may optionally also be present in any of the other discussed embodiments, such as the embodiments show in Figure 1 or Figure 2.

It is noted that the functional components that are present in the embodiment of Figure 2 in combination, are operationally largely independent. In further variants, only one or some of the additional functional units may therefore be present.

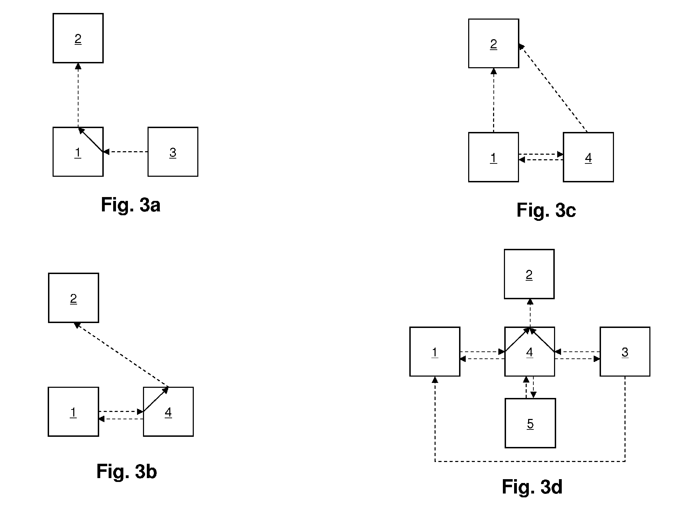

Figure 3a schematically shows a further exemplary ambulatory infusion system. The system includes infusion pump device 1 and earphone device 2 as generally discussed above. The system additionally includes a further device in form of a continuous blood glucose monitor (CGM) 3. CGM 3 is typically realized on basis of an electro-chemical sensor that is placed on a subcutaneous electrode as generally known in the art. CGM 3 may be made as fully disposable device with a lifetime in the range of typically some days up to about two weeks, or may be realized in a modular way with a disposable sensor module and a durable electronics module that is designed for a longer lifetime of, e.g. several months or even generally unlimited (open) lifetime. While not being essential, CGM 3 is in the following considered to be a pure measurement device without user interface.

In the embodiment of Figure 3a, infusion pump device 1 and earphone device 2 generally operate in the same way as discussed above with reference to Figs 1, 2. In addition, however, pump communication unit 15 is designed to wirelessly couple to a wireless CGM communication unit (not separately shown) of CGM 3. In operation, blood glucose values that are measured by CGM 3 are transmitted form CGM 3 to infusion pump device 1. Infusion pump device 1, in particular pump control unit 12, is designed to further process the blood glucose data and to provide corresponding information e.g. in form of measured glucose values, averaged glucose values, maximum and/ or minimum values, glucose-vs.-time graphs, trend information etc. in form of user signalization. Alternatively or additionally to glucose measurement values being processed by pump control unit 12, CGM 3 may be designed to do some or all of the processing. CGM 3 typically further includes signal conditioning and processing circuitry, such as impedance converters, amplifiers, filters, etc. Inputting information into CGM 3, which may, e.g. be required for calibration purposes, may be carried out via infusion pump input unit 17, thereby additionally serving as CGM input unit. Additionally or alternatively, CGM 3 may have its own user interface.

Pump communication unit 12 is further designed to receive at least one CGM message from CGM 3 and to relay it to earphone communication unit 21. Alternatively, CGM 3 may be designed to transmit glucose related data, such as measured "raw" blood glucose values, to infusion pump device 1 where the CGM message to be indicated is generated and transmitted to earphone device 2. After receiving a CGM message, earphone device 2 is designed to actuate the earphone audio transducer 22 in the same way as discussed above in the context of pump messages.

A CGM message may, e.g., be or include a current or averaged blood glucose value, blood glucose trend information, a blood glucose alert indicating a measured or predicted blood glucose value exceeding an upper blood glucose threshold or falling below a lower blood glucose threshold. A CGM message may further be or include related to operation of CGM 3 and indicate, e.g., the requirement for calibration data being provided from a separate blood glucose measurement device, a requirement for the sensor module or the whole CGM to be replaced, a CGM malfunction, a CGM power source being largely depleted, etc.

While Figure 3a exemplary shows an ambulatory infusion system including CGM 3, further devices, such as a remote controller may be additionally or alternatively be present and transmit messages to earphone device 2 via infusion pump device 3 in substantially the same way.

For an ambulatory infusion system that includes a number of distinct and separate devices, relaying further device messages via infusion pump device 1 or generating further device messages in the infusion pump device has the advantage that the infusion pump device as particularly safety-relevant and critical component of the overall system is the major canter of all activity receives all information that is transmitted to the earphone device, e.g. for record keeping, consistency checking, and the like. In addition, it is sufficient for earphone device 2 to only communicate with infusion pump device 1 via point-to-point connection.

Figure 3b schematically shows a further exemplary ambulatory infusion system. The system includes infusion pump device 1 and earphone device 2 as generally discussed above. The system further includes remote controller 4. Remote controller 4 is designed for controlling operation of infusion pump device 1 via a remote controller input unit (not separately shown) and a remote controller output unit (not separately shown). The remote controller input unit may be designed in a similar way to pump input unit 17. The remote controller output unit may include an audio transducer similar to pump audio transducer 16b and/ or non-audio indication units. Remote controller 4 may further include a blood glucose measurement device (not referenced) for single-spot blood glucose measurements. Where not mentioned otherwise, the overall design of remote controller 4 may be similar to the commercial ACCU-CHEK® Aviva Combo device by Roche diagnostics, or similar devices.

Remote controller 4 is designed to generate at least one remote controller message and to transmit the at least one remote controller message to earphone communication unit 21 via a remote controller communication unit (not separately shown). After receiving a remote controller message, earphone device 2 is designed to actuate the earphone audio transducer 22 in the same way as discussed above in the context of pump messages. A remote controller message may be therapy-related, such as a blood glucose value measured by a blood glucose measurement device of remote controller 4, or be related to operation of remote controller 4 itself.

The remote controller communication unit is further designed to receive the at least one pump message from infusion pump device 1 and to relay it to earphone communication unit 21. In this embodiment, pump communication unit 15 and earphone device communication unit 21 may not be designed for direct wireless communication.

A system design according to Figure 3b has the advantage that the particularly therapy-critical infusion pump device 1 and especially pump control unit 12 can be tailored to functionality that is essential for drug infusion, thereby reducing complexity, required computing and processing performance, and power consumption of infusion pump device 1. Like in the embodiment of Figure 3a, only a single point-to-point connection is require for earphone device 2. Providing pump messages via earphone device 2, however, requires remote controller 4 to be present and operable (i.e. switched on and communication links being established or ready for establishment with both infusion pump device 1 and earphone device 2). Therefore, this type of embodiment is suite for earphone indication of critical messages mainly if infusion pump device 1 includes an additional pump audio transducer.

Figure 3c schematically shows a further exemplary ambulatory infusion system. Similar to the embodiment of Figure 3a, an infusion pump device 1, an earphone device 2 and an remote controller 4 are present. In contrast to Figure 3a, however, earphone communication unit 21 is designed for direct and independent communication with infusion pump communication 15 and the remote controller communication unit. Pump messages and remote controller messages are separately transmitted to earphone device 2 where earphone audio transducer 22 is actuated in accordance with the received message.

While being somewhat more complex with respect to inter-device communication as compared to the previously described embodiments, this type of embodiment is particularly flexible and still allows providing audio message from either of infusion pump device 4 or remote controller 4 (and/ or any other further device) via earphone device 2 if the other of infusion pump device or remote controller 4 (and/ or any other further device) is not present or not in an operative state.

Figure 3d schematically shows a further exemplary ambulatory infusion system. In this embodiment, infusion pump device 1, earphone device 2, CGM 3, and remote controller 4 are present. In addition, optional remote computer 5, such as a standard Personal Computer (PC) is shown in communication with remote controller 4 that may be used for long-term therapy documentation and advanced therapy evaluation purposes and/ or serve as comfortable user interface of configuration of any of the other devices.

Like in the embodiment of Figure 3b, remote controller 4 is designed to generate remote controller messages that are transmitted to earphone device 2. Also like in the embodiment of Figure 3b, remote controller 4 is designed to receive pump messages from infusion pump device 1 and to relay them to earphone device 2.

In addition, remote controller 4 is designed to receive CGM messages from CGM 3 and to relay the CGM messages to earphone device 2. In the embodiment of Figure 3d, remote controller 4 accordingly serves as central device that is designed to communicate with all other devices of the system that may generate messages and relay those audio messages to earphone device 2.

In some embodiments, glucose measurements that may be provided via a blood glucose measurement device of remote controller 4, or CGM 3 may be used to influence and thereby modify operation of infusion pump device 1. In some of those embodiments, infusion pump device 1 is designed to operate, together with CGM 3, in a closed control loop where insulin infusion by infusion pump device 1 is adjusted in a substantially continuous way in order to maintain the diabetic's blood glucose level in a desired target range. For such a closed-loop control, a number of control algorithms with different levels of complexity are known in the art. Alternatively, blood glucose values that are substantially continuously measured by CGM 3 may be used to temporarily suspend or reduce basal insulin infusion by infusion pump device 1 in case of the diabetic's blood glucose level falling below a pre-set blood glucose threshold. Corresponding algorithms are disclosed, e.g., in the WO 2006/ 083831 A1.

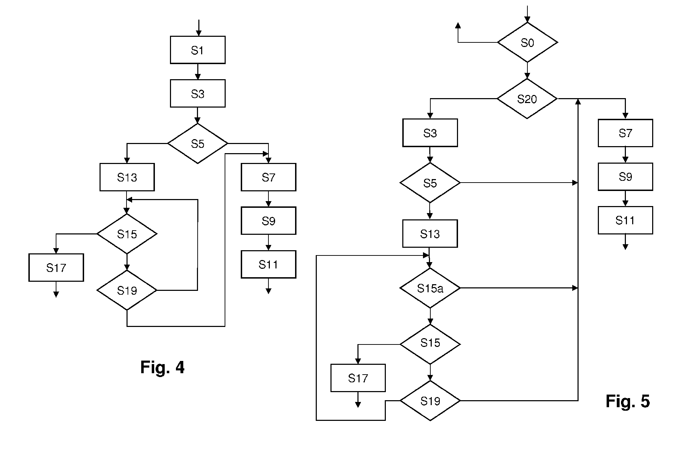

Figure 4 schematically shows the major steps of an exemplary operational flow for providing audio messages to a user of an ambulatory infusion system that is designed in accordance with Figure 2.

In step S1, a pump message is generated by infusion pump device 1, typically by pump control unit 12. The pump message may be an alert or alarm or may be an information message.

In step S3, a communication channel between pump communication unit 15 and earphone communication unit 21 is established or it is verified that such a communication channel has been established before and is operable. In dependence of the result of step S3, operational flow branches in following step S5.

If, in step S3, a communication channel could not be established or an operational state of such a communication channel could not be verified, pump audio transducer 16b is actuated in step S7 to provide a user signalization in accordance with the pump message. In following step S9, reception of an acknowledgement user input is awaited from pump input unit 17. After receiving such acknowledgement user input, actuation of pump audio transducer 16b is stopped in following step S11, thereby stopping the signalization.

If, in step S3, a communication channel could be established or an operational state of such a communication channel could be verified, operational flow continues with step S13 instead of step S7. In step S13, the pump message is transmitted from pump communication unit 15 to earphone communication unit 21 and earphone audio transducer 22 is actuated to provide a user signalization in accordance with the pump message. At the same time, an acknowledgement timer is started.

In following step S15, reception of a corresponding acknowledgement user input is tested. The acknowledgement user input may be provided by pump input unit 17 or earphone input unit 24. If the acknowledgement user input is received, actuation of earphone audio transducer 22 is stopped in following step S17, thereby stopping the signalization. If the acknowledgement user input is provided via pump input unit 17, a corresponding stopping command may be generated by pump control unit 12 and transmitted to earphone device 2. If the acknowledgement user input is provided via earphone input unit 24, earphone device 2 may automatically stop actuation of earphone audio transducer 22 and favourably send a corresponding acknowledgement confirmation signal to infusion pump device 1. Alternatively, however, the acknowledgement user input may first be relayed to infusion pump device 2, where pump control unit 12 generates the stopping command that is subsequently transmitted to earphone device 2. While involving some additional communication effort, it ensures that pump control unit 12 is always in control of the signalization.

If no acknowledgement user input is received in step S15, expiry of the acknowledgement time interval is tested in following step S19. If the acknowledgement time interval has not yet expired, operational flow returns to step S15 and a reception of the acknowledgement user input is further awaited.

If no acknowledgement user input is received within the acknowledgement time interval, operational flow branches in step S19 to step S7 and signalization is provided via pump audio transducer 16b as described before.

In the above-given description, it is assumed that the pump message as generated in step S1 requires user confirmation. If this is not the case, e.g. for an information message, the acknowledgement-related steps may be omitted.

In addition to actuation pump audio transducer 16b and/ or earphone audio transducer 24, non-audio indication unit 16a may optionally be actuated. In case of non-audio indication unit 16a including, e.g. a display, the display e.g., may provide an alarm or alert message and/ or information such as the liquid volume remaining in drug container 13.

Figure 5 schematically shows the major steps of a further exemplary operational flow for providing audio messages to a user of an ambulatory infusion system that is designed in accordance with Figure 2. In this embodiment, the exemplary infusion system is designed to different signalization priorities. For clarity reasons, only two signalization priorities, namely "low priority" and "high priority" are considered here. The concept and the operational flow, however may be adopted to a more differentiated scheme in a straightforward way.

In Figure 5, operational steps that are identical to the embodiment of Figure 4 carry identical step numbers and are not discussed in the following in detail.

The operational flow of Figure 5 is carried out substantially continuously during normal operation of the ambulatory infusion system. In an initial state, it is assumed that no signalization status is activated and no pump message is being provided.

In step S0, it is tested whether any signalization has been activated since last carrying out the operations of Figure 5. If this is not the case, operational flow continues as before.

If it is determined in step S0 that at least one signalization status has been activated, operational flow branches in step S20 in dependence of the signalization priority. In case of "high priority", operational flow continues with steps S7 - S11 where pump audio transducer 16b is activated to provide signalization in a accordance with a pump message associated with the activated signalization status as described above with reference to Figure 4. While not shown in Figure 5, the pump message may additionally be transmitted to earphone device 2.

In the alternative case of "low priority", steps S3 - S19 are generally carried out in the same way as discussed above with reference to Figure 4 for providing signalization via earphone device 2, with the following deviations.

Prior to t testing reception of a corresponding acknowledgement user input in step S15, step S15 a is carried out where it is tested while providing the "low priority" pump message via earphone device 2, a further "high priority alert" has occurred. In this case, operational flow also branches to step S7 to provide signalization in accordance with the "high priority" pump message via pump audio transducer 16b. While not explicitly shown, signalization of the "low priority" pump message via earphone device 2 is favourably stopped in this case or earphone device 2 is controlled to also provide signalization in accordance with the "high priority" pump message.

Since step S15a is substantially continuously carried out while awaiting the acknowledgement user input, it is ensured that a "high priority" pump message is always signalized.

Along with ending a signalization in either of step S11 or step S7, the corresponding signalization status is deactivated.

## Claims

1. Ambulatory infusion system, including
- an infusion pump device (1) including a pump unit (11) and an electronic pump control unit (12), the pump control unit (12) being operatively coupled to the pump unit (11) to control the pump unit (11) to continuously infuse a liquid drug into a patient's body, the infusion pump device (1) further including a wireless pump communication unit (15), wherein the infusion pump device (1) is designed to generate a pump message to be signalized to a user, and
- a signalization device for providing user signalization in accordance with the pump message,
wherein the signalization device includes an earphone device (2), the earphone device (2) including an earphone audio transducer (22), the earphone audio transducer (22) being designed for attachment to a user's head in direct acoustic coupling to an ear of the user, the earphone device (2) further including a wireless earphone communication unit (21),
wherein the infusion pump device (1) is designed to transmit the pump message, via the pump communication unit (15), to the earphone communication unit (21), and wherein the earphone device (2) is designed to receive the pump message via the earphone communication unit (21) and to actuate the earphone audio transducer (22) to produce a pump audio message in accordance with the pump message.

2. Ambulatory infusion system according to claim 1, further including at least one further device (3, 4), the at least one further device (3, 4) including a wireless further device communication unit, the further device communication unit being designed for wireless coupling, in particular direct wireless coupling, with at least one of the pump communication unit (15) and the earphone communication unit (21).

3. Ambulatory infusion system according to claim 2, wherein the at least further device (3, 4) includes either of a cell phone, a pump remote controller (4), a blood glucose measurement device, and a continuous blood glucose monitor (3).

4. Ambulatory infusion system according to either of claim 2 or claim 3, wherein the further device communication unit is designed to receive the pump message from the pump communication unit (15) and to relay the pump message to the earphone communication unit (21).

5. Ambulatory infusion system according to either of claim 2 to claim 4, wherein the at least one further device is designed
- to generate at least one further device message and to transmit the at least one further device message, via the further device communication interface, to the pump communication interface (15), and the pump communication interface (15) is designed to receive the at least one further device message and to relay the at least one further device message to the earphone communication interface (21), or
- to transmit data, via the further device communication interface, to the pump communication interface (15) and the infusion pump device (1) is designed to receive, via the pump communication interface (15), the data and to generate the at least one further device message based on the transmitted data, and to transmit the at least one further device message, via the pump communication interface (15), to the earphone communication interface (21).

6. Ambulatory infusion system according to any of the preceding claims, wherein the earphone device (2) includes a earphone input unit (24), and wherein the pump communication unit (15) and the earphone communication unit (21) are designed for transmission of a user input that is entered via the earphone input unit (24) from the earphone device (2) to the infusion pump device (1).

7. Ambulatory infusion system according to claim 6, wherein the user input includes an information request input, and the pump control unit (12) is designed to generate the pump message in response to receiving, via the pump communication unit (15), the information request input.

8. Ambulatory infusion system according to any of the preceding claims, wherein the signalization device includes a non-audio indication unit (16a) and wherein the infusion pump device (1) is designed to actuate the non-audio indication unit (16) in accordance with the pump message additionally or alternatively to transmitting the pump message to the earphone communication unit (21).

9. Ambulatory infusion system according to any of the preceding claims, wherein the infusion pump device (1) is designed to generate the pump message alternatively as a number of distinct pump messages.

10. Ambulatory infusion system according to claim 9, wherein each of the number of distinct pump messages is associated with a corresponding signalization status that may be independently activated or deactivated, with an activated signalization status indicating that a signalisation is to be provided in accordance with the corresponding pump message, and wherein each of the number of distinct pump messages is associated with a corresponding signalization priority, and wherein the ambulatory infusion system is designed to determine, within a number of pump messages having an activated associated signalization status, a priority pump message of highest signalization priority, and to control the signalization device (16a, 16b, 22) to provide user signalization in accordance with the priority pump message.

11. Ambulatory infusion system according to any of the preceding claims, wherein the infusion pump device (1) includes a pump audio transducer (16b) and wherein the pump control unit (12) is designed to actuate the pump audio transducer (16b) in accordance with the pump message additionally or alternatively to transmitting the pump message to the earphone communication unit (21).

12. Ambulatory infusion system according to claim 10 and claim 11, wherein the pump control unit (12) is designed to selectively actuate the pump audio transducer (16b) in accordance with the pump message based on the pump message priority associated with the pump message.

13. Ambulatory infusion system according to either of claim 11 or claim 12, wherein the infusion pump device (1) is designed to determine whether a communication channel between the pump communication unit (15) and the earphone communication unit (21) is established and to transmit the pump message to the earphone communication unit (21) without activating the pump audio transducer (16b) if communication between the pump communication unit (15) and the earphone communication unit (21) is established and to activate the pump audio transducer (16b) if communication between the pump communication unit (15) and the earphone communication unit (21) is not established.

14. Ambulatory infusion system according to either of claims 11-13, wherein the infusion pump device (1) is designed to transmit the pump message, via the pump communication unit (15), to the earphone communication unit (21) without activating the pump audio transducer (16b) and to activate the pump audio transducer (16b) if no acknowledgement user input is received within a following acknowledgement time interval.

15. Ambulatory infusion system according to either of claim 6 or 7 and according to claim 14, wherein the user input includes the acknowledgement user input.

16. Ambulatory infusion system according to any of the preceding claims, including a voice message unit (23), the voice message unit (23) being designed to receive the pump message and to generate, based on the pump message, a corresponding pump voice message, and to actuate the earphone audio transducer (21) to produce the pump voice message.

17. Ambulatory infusion system according to claim 16, wherein the voice message unit (23) is included in either of the infusion pump device (1) or the earphone device (2).

18. Ambulatory infusion system according to either of claim 16 or 17, wherein the pump voice message is indicative of a situation requiring urgent user interaction, in particular a blockage or leakage of an infusion line (14), an empty liquid drug container (13), an empty power source, or a electric and/ or mechanic failure of the ambulatory infusion system.

19. Ambulatory infusion system according to either of the preceding claims, wherein the signalization device further includes a head-mounted display apparatus (25) and the infusion pump device (1) is designed to generate at least one further pump message and to transmit the at least one further pump message, via the pump communication unit, to the head-mounted display apparatus (25),
wherein the head-mounted display apparatus (25) is designed to receive the wirelessly transmitted further pump message and to actuate an image-forming device of the head-mounted display device to produce a pump display message in accordance with the at least one further pump message.

20. Infusion pump device (1), the infusion pump device (1) being designed for use in an ambulatory infusion system according to any of the preceding claims.
